# EUROPÄISCHE PATENTSCHRIFT

(11) **EP 2 156 193 B1**
(45) Veröffentlichungstag und Bekanntmachung des Hinweises auf die Patenterteilung: **01.09.2010**
(21) Anmeldenummer: 08734458.6
(22) Anmeldetag: 01.04.2008
(51) Int. Cl.: G01N 33/84

(54) **OPTISCHES MESSVERFAHREN ZUR ERMITTLUNG DES PH-WERTS EINES MEDIUMS UNTER ANWENDUNG VON AGELADINE A ALS FLUORESZIERENDEM PH-WERT-INDIKATOR**
OPTICAL MEASUREMENT METHOD FOR DETERMINING THE PH OF A MEDIUM USING AGELADINE A AS A FLUORESCENT PH INDICATOR
PROCEDE DE MESURE OPTIQUE POUR DETERMINER LE pH D'UN MILIEU AU MOYEN D'AGELADINE A UTILISEE COMME INDICATEUR DE pH FLUORESCENT

(30) Priorität: 28.05.2007 DE 102007024985; 15.07.2007 DE 102007034886
(43) Veröffentlichungstag der Anmeldung: 24.02.2010
(73) Patentinhaber: Stiftung Alfred-Wegener-Institut für Polar- und Meeresforschung, 27570 Bremerhaven (DE)
(72) Erfinder: BICKMEYER, Ulf-Georg, 27572 Bremerhaven (DE); KLINGS, Karl-Walter, 27498 Helgoland (DE); GRUBE, Achim, 88471 Laupheim (DE); KÖCK, Matthias, 60439 Frankfurt (DE)
(86) Internationale Anmeldenummer: PCT/DE2008/000570
(87) Internationale Veröffentlichungsnummer: WO 2008/145080

(56) Entgegenhaltungen:
- DE-A1-102004 002 885
- DE-C2- 19 681 363
- FUJITA MASAKI ET AL: "Ageladine A: an antiangiogenic matrixmetalloproteinase inhibitor from the marine sponge Agelas nakamurai" JOURNAL OF THE AMERICAN CHEMICAL SOCIETY, AMERICAN CHEMICAL SOCIETY, WASHINGTON, DC.; US, US, Bd. 125, Nr. 51, 24. Dezember 2003 (2003-12-24), Seiten 15700-15701, XP008094136 ISSN: 0002-7863 in der Anmeldung erwähnt

## Beschreibung

Die Erfindung bezieht sich auf ein optisches Messverfahren zur Ermittlung des pH-Werts eines Mediums durch
- Zugabe eines fluoreszierenden pH-Wert-Indikators in das Medium,
- Fluoreszenzanregung des pH-Wert-Indikators durch Bestrahlung des pH-Wert-Indikators mit Licht zumindest einer ausgewählten Wellenlänge und
- Detektion der emittierten Fluoreszenzintensitäten des pH-Wert-Indikators als Maß für den pH-Wert des Mediums.

Die Wasserstoffkonzentration oder der pH-Wert ist ein extrem wichtiger Parameter in biologischen und chemisch/technischen Systemen. Viele chemische und biologische Reaktionen erfordern eine genaue Regelung des pH-Werts für einen korrekten Ablauf. Beispielsweise findet ein komplexes natürliches Verfahren zur Regelung des pH-Werts in menschlichem Blut statt, das normalerweise einen pH-Wert von ungefähr 7,4 hat. Veränderungen von selbst einigen Zehnteln einer pH-Werteinheit können bereits ernste Krankheitszustände zum Grund haben oder verursachen. Obwohl eine Vielfalt von Techniken entwickelt worden ist, um den pH-Wert zu messen, basieren diese allgemein auf entweder elektrochemischen oder optischen Prinzipien. Ein Standard-Labormessgerät für den pH-Wert umfasst beispielsweise eine Standardelektrode mit bekanntem Potenzial, eine spezielle Glaselektrode, die das Potenzial in Abhängigkeit von der Konzentration der Wasserstoffionen im Medium ändert, in das sie eintaucht, und ein Potenziometer, welches das Potenzial zwischen den beiden Elektroden misst, aus dem ein numerischer pH-Wert ermittelt wird. Verfahren dieser Art sind jedoch nicht sehr gut für Messungen in intakten biologischen Systemen geeignet, da eine Messelektrode eingeführt werden muss.

Im Rahmen von optischen Messungen werden pH-Wert-Indikatoren verwendet, bei denen es sich um Farbstoffe handelt, deren detektierbare optische Eigenschaften, wie Extinktion (Absorption) oder Fluoreszenz, sich mit Veränderung des pH-Werts ebenfalls ändern. Somit zeigen pH-Wert-Indikatoren durch ihren Farbton und ihre Farbintensität den aktuellen pH-Wert der Lösung an. Die größte Empfindlichkeit von Indikatoren auf kleine Änderungen des pH-Werts liegt vor, wenn die Gleichgewichtskonstante (pKₐ) zwischen den sauren und basischen Formen des Indikators nahe dem Wert des zu untersuchenden Mediums, in der Regel eine Lösung, liegt.

Als breite Verallgemeinerung werden optische Messungen des pH-Werts als den elektrochemischen Techniken unterlegen angesehen, hauptsächlich weil von der Wasserstoffionenkonzentration verschiedenen Faktoren wie Temperatur, Ionenstärke und Proteinkonzentration die Farbstoffe beeinflussen und die pH-Wertmessungen stören können. Dennoch haben optische Techniken erhebliche Vorteile, wenn Kosten und Größe eine Rolle spielen und besonders, wenn in lebenden Zellen und Geweben der pH-Wert gemessen werden soll, in die keine Messsonde eingeführt werden kann. Messungen von pH-abhängiger Emissionsintensität in einzelnen Zellen mit einer einzigen Anregungswellenlänge leiden unter Ungenauigkeiten, die mit der Farbstoffkonzentration, Photobleichung des Farbstoffs, Messzellendicke oder Weglänge zusammenhängen. Eine Lösung des Problems der Farbstoffkonzentration besteht in der Bestimmung des Verhältnisses der Fluoreszenzintensität bei einer festen Wellenlänge mit Anregung bei einer pH-empfindlichen Wellenlänge zu der Fluoreszenzintensität bei der gleichen Wellenlänge mit Anregung bei einer relativ pH-unempfindlichen Wellenlänge. Dieses Verfahren wird üblicherweise verwendet, um den pH-Wert im Inneren von Zellen mit Fluoreszenzderivaten zu schätzen, und ist praktisch geeignet für Suspensionen von Zellen und in homogenen Flüssigkeiten wie Medien in einem Forschungsfluorometer oder Mikroskop.

### STAND DER TECHNIK

Ein optischer Biosensor ist aus der DE 39 23 921 A1 bekannt, bei dem ein chemische Reaktionen katalysierendes Enzym an einen pH-Wert-Indikator gebunden ist. Bei dem Indikator kann es sich um einen Fluoreszenzfarbstoff, insbesondere auf der Basis von Cumarin, handeln. Durch messbare Änderung der Fluoreszenzintensität des Farbstoffs kann eine Änderung des pH-Wertes während der chemischen Reaktion festgestellt werden. Aus der DE 600 13 613 T2 ist ein Kulturmedium für den Nachweis und die zahlenmäßige Bestimmung von Mikroorganismen bekannt, das einen pH-Wert-Indikator umfasst, der chemisch an eine hydrophile Substanz mit hohem Molekulargewicht gebunden ist. Somit wird ein wasserlöslicher pH-Wert-Indikator mit Ballast zur Verfügung gestellt, der gleichzeitig als Nährstoffgel das hydratisierte Wachstum der Mikroorganismen unterstützt. Ein Wachstumsnachweis wird durch Nachweis der Fluoreszenzveränderung des pH-Wert-Indikators, der Carboxyphenolrot umfassen kann, im Kulturmedium erbracht. Die DE 101 52 994 A1 offenbart ein optisches Verfahren zur gleichzeitigen Bestimmung von pH-Wert und Gelöstsauerstoff. Dazu werden zwei fluoreszierende pH-Wert-Indikatoren, beispielsweise auf der Basis von Ruthenium, in einer gemeinsamen Matrix im Medium eingesetzt.

Aus der US 4.945 171 A ist ein pH-Wert-Indikator auf der Basis von fluoreszierenden Xanthinfarbstoffen bekannt. Hierbei werden zwei Emissionsmaxima bei der Anregung mit nur einer Wellenlänge mit besonderer Selektivität für die unabhängige Anregung von Säure- und Baseform bei entweder einer einzigen oder zwei Wellenlängen und die daraus resultierenden pH-Wert-abhängigen Absorptions- oder Fluoreszenzanregungsspektren gemessen. Aus der DE 196 81 363 C2**,** von der die vorliegende Erfindung als nächstliegendem Stand der Technik ausgeht, ist ein pH-Wert-Indikator auf der Basis von fluoreszierenden Carbazinfarbstoffen und Derivaten bekannt. Gegenüber den Xanthinfarbstoffen zeigen die Carbazinfarbstoffe eine höhere Fluoreszenz, eine größeren Stabilität, eine geringere Temperaturempfindlichkeit und höhere Stokes-Verschiebungen. Außerdem sind die Carbazinfarbstoffe besser auf einem festen Träger zu immobilisieren. Es wird ein Verfahren zum Anzeigen des pH-Werts einer Lösung als Medium offenbart, bei dem der pH-Wert-Indikator in die Lösung gegeben und mit Licht einer ausgewählten Wellenlänge in Kontakt gebracht wird, um den Carbazinfarbstoff zur Fluoreszenz anzuregen, die Intensität der Fluoreszenz bei zwei verschiedenen Wellenlängen gemessen wird, das Verhältnis der Fluoreszenzintensitäten bei den zwei gewählten Wellenlängen berechnet wird und das Verhältnis mit einer vorgegebenen Beziehung für solche Verhältnisse mit dem pH-Wert korreliert wird.

Weitere Fluoreszenz-Farbstoffe, die bereits zu Messung des pH-Werts eingesetzt werden, sind beispielsweise die Farbstoffe BCECF und DNP-160. Weitere molekulare Sonden aus der pH-Wert-Indikator-Familie sind beispielsweise der nachfolgenden Tabelle zu entnehmen (sortiert nach abnehmender Gleichgewichtskonstante pKₐ, Tabellen entnommen aus http://probes.invitrogen.com/handbook/tables/0361.html, Stand 27.05.2007). Deutlich zu erkennen ist der jeweils geringe Umfang des detektierbaren pH-Wert-Bereichs.

| **VORGÄNGER- FLUOROPHOR** | **PH-BEREICH** | **TYPISCHE MESSUNG** |
|---|---|---|
| SNARF Indikatoren | 6,0-8,0 | Emissionsrate 580/640 nm |
| HPTS (Pyranin) | 7,0-8,0 | Anregungsrate 450/405 nm |
| BCECF | 6,5-7,5 | Anregungsrate 490/440 nm |
| Fluoreszeine und Carboxyfluoreszeine | 6,0-7,2 | Anregungsrate 490/450 nm |
| LysoSensor grün DND-189 | 4,5-6,0 | einzelne Emission 520 nm |
| Oregon grüne Farbstoffe | 4,2-5,7 | Anregungsrate 510/450 nm oder 490/440 nm |
| LysoSensor gelb/blau DND-160 | 3,5-6,0 | Emissionsrate 450/510 nm |

Bei dem bioaktiven marinen Naturstoff Ageladine A (chemische Formel C₁₀H₇N₅Br₂) handelt es sich um ein Pyrrol-Imidazol Alkaloid, das beispielsweise aus Schwämmen der Gattung *Agelas* isoliert werden kann (vergleiche **VERÖFFENTLICHUNG I** von M. Fujita et al.: "Ageladine A: An Antiangiogenetic Matrixmetalloproteinase Inhibitor from Marine Sponge Agelas nakamurai", J. Am. Chem. Soc. 2003, 125, 15700-15701 und Supporting Information S.I. 1-15). Ageladine A kann mittlerweile auch vollständig synthetisiert werden (vergleiche **VERÖFFENTLICHUNG II** von M. Meketa et al.: "Total Synthesis of Ageladine A, an Angiogenesis Inhibitor from the Marine Sponge Agelas nakamurai" Org. Lett. 2006, 8, 7, 1443-1446, **VERÖFFENTLICHUNG III** VON S. Shengule et al.: "Concise Total Synthesis of the Marine Natural Product Ageladine A", Org. Lett. 2006, 8, 18, 4083-4084 und **VERÖFFENTLICHUNG IV** von M. Meketa et al.: "A New Total Synthesis of the Zinc Matrixmetalloproteinase Inhibitor Ageladine A Featuring a Biogenetically Patterned 6π-2-Azatriene Electrocyclization", Org. Lett. 2007, 9, 5, 853-855). Ageladine A steht somit der Öffentlichkeit in unbegrenztem Umfang zur Verfügung. Bei wissenschaftlichen Untersuchungen von Ageladine A mit Messungen zur zellulären Wirkung von marinen Naturstoffen zeigt Ageladine A eine störende Eigenfluoreszenz (vergleiche **VERÖFFENTLICHUNG V** von U. Bickmeyer et al.: "Disturbance of Voltage Induced Cellular Calcium Entry by the Marine Pyrrole-Imidazole Alkaloids", doi:10.1016/j.toxicon.2007.04.015). Ageladine A hat nach UV-Anregung eine ausgeprägte Fluoreszenz im Grünbereich (siehe **VERÖFFENTLICHUNG I**).

Die DE 10 2004 002 885 B4 offenbart eine Reihe neuer bioaktiver Verbindungen aus der Klasse der Pyrrolalkaloide. Dabei sind Meeresschwämme eine reichhaltige Quelle an Pyrrolalkaloiden, einer Gruppe von Naturstoffen, die sich durch ihre strukturelle Vielfalt und interessante biologische Aktivitäten auszeichnet. Genannt werden auch Pyrrol-Imidazol Alkaloide, gewonnen aus verschiedenen karibischen Schwammarten der Gattung *Agelas,* mit einer selektiven antihistaminischen Wirkung und Agelongin (Strukturformel 22) aus *Agelas longissima* mit einer antiserotonergen Wirkung an Funduspräparationen von Rattenmägen. Alle genannten bioaktiven Verbindungen werden ausschließlich für medizinische Zwecke eingesetzt, insbesondere zur Bekämpfung von neurodegenerativen Erkrankungen.

### AUFGABENSTELLUNG

In Anbetracht der vorangehenden Ausführungen zu bekannten optischen Messverfahren zur Ermittlung des pH-Werts eines Mediums ist die **AUFGABE** für die vorliegende Erfindung darin zu sehen, ein verbessertes gattungsgemäßes Messverfahren anzugeben, bei dem ein zu den bekannten fluoreszierenden pH-Wert-Indikatoren alternativer fluoreszierender pH-Wert-Indikator verwendet wird, der eine verminderte Empfindlichkeit gegenüber potenziell störenden Faktoren und einen großen pH-Wert-Messbereich zeigt. Insbesondere soll eine wesentlich verbesserte Messleistung in biologischen Systemen, von denen die meisten im Bereich von pH5 bis pH9 arbeiten, gewährleistet sein

Die LÖSUNG für diese Aufgabe ist dem Verfahrensanspruch zu entnehmen. Vorteilhafte Weiterbildungen des Verfahrens sind in den Unteransprüchen aufgezeigt und werden im Folgenden im Zusammenhang mit der Erfindung näher erläutert.

Das erfindungsgemäße optische Messverfahren ist dadurch gekennzeichnet, dass ein zu den bekannten fluoreszierenden pH-Wert-Indikatoren alternativer fluoreszierender pH-Wert-Indikator auf Basis von Ageladine A verwendet wird. Bei Ageladine A handelt es sich um einen bekannten marinen Naturstoff in Form eines Pyrrol-Imidazol Alkaloids, der im Stand der Technik ausschließlich zu medizinischen Zwecken eingesetzt wird. Ageladine A kann sowohl aus marinen Schwämmen gewonnen als auch synthetisch hergestellt werden, wie oben bereits ausgeführt. Durch die beschriebenen Totalsynthesen ist es somit ohne weiteres möglich, größere Mengen von Ageladine A bereitzustellen, ohne dabei auf marine Schwämme zurückgreifen zu müssen. Zur Nutzung von Ageladine A im Rahmen der vorliegenden Erfindung hat sich herausgestellt, dass die auftretende Eigenfluoreszenz durch Anregungsverstärkung mittels Bestrahlung mit Licht im UV-Bereich zwischen 315 nm und 400 nm Wellenlänge für Messzwecke einsetzbar ist. Ageladine A zeigt dann eine ausgeprägte Fluoreszenz im Grünbereich zwischen 490 nm und 575 nm Wellenlänge. Bei der Verwendung von Ageladine A als fluoreszierendem pH-Wert-Indikator können pH-Wert-Änderungen des zu untersuchenden Mediums zwischen pH4 und pH9 durch eine veränderliche Fluoreszenzintensität in der Weise zuverlässig optisch angezeigt werden, dass mit sinkendem pH-Wert die emittierte Fluoreszenzintensität ansteigt. Der von Ageladine A anzeigbare pH-Wert-Bereich ist ungewöhnlich groß für einen fluoreszierenden pH-Wert-Indikator und die Färbungen erweisen sich als zeitlich sehr stabil.

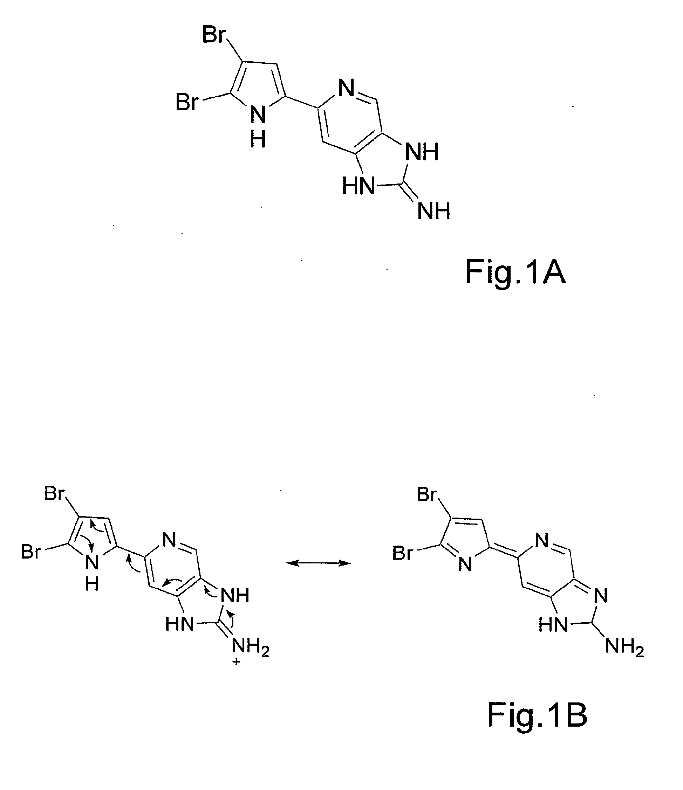

Die fluoreszierenden Eigenschaften von Molekülen sind auf ihre chemische Struktur zurückzuführen. Dabei sollten folgende, allgemeine Punkte durch die chemische Molekülstruktur erfüllt sein: aromatisches bzw. delokalisiertes π-Elektronensystem, Chromophor, starrer und planarer Molekülaufbau. Alle diese Punkte werden von der Molekülstruktur von Ageladine A erfüllt. Die Struktur weist ein aromatisches System (Pyrrol, Pyridin) sowie das π-Elektronensystem des Guanidins auf. Durch die basische Guanidinstruktur ist eine Protonierung in sauren Medien an dieser Stelle sehr wahrscheinlich. Die dabei auftretende positive Ladung kann dabei über das gesamte Molekül delokalisiert werden, was Voraussetzung für ein chromophores System ist. Die formulierte Grenzstruktur weist eine starre bzw. planare Molekülstruktur auf. Damit können die fluoreszierenden Eigenschaften und auch pH-Wertabhängige Änderungen in der Fluoreszenz erklärt werden (vergleiche Figuren 1A, 1B des speziellen Beschreibungsteil).

Überraschend hat sich weiterhin herausgestellt, dass Ageladine A eine besonders hohe Empfindlichkeit der emittierten Fluoreszenzintensität im physiologisch relevanten Bereich zwischen pH6 und pH8 aufzeigt. Somit kann Ageladine A als pH-Wert-Indikator besonders gut für physiologische pH-Wert-Messungen eingesetzt werden. Vorteilhafterweise können entnommene physiologische Proben (in vitro) aber auch lebende Zellen, Gewebe und ganze Organismen (in vivo) mit Ageladine A als fluoreszierendem pH-Wert-Indikator inkubiert und durch die Fluoreszenz im Grünbereich auf diese Art gefärbt werden. Dies ermöglicht Messungen des pH-Werts in vivo und in vitro in einem weiten Bereich und damit die Erkennung saurer Gewebe in Organismen durch seine Fluoreszenz-Eigenschaften. Die Fluoreszenz im Grünbereich nimmt mit sinkendem pH-Wert deutlich zu. Die intensiven Färbungen erweisen sich als stabil über Stunden bis zu Tagen.

Mit dem Verfahren nach der Erfindung unter der Anwendung von Ageladine A als fluoreszierendem pH-Wert-Indikator können somit pH-Wert-Messungen von Lösungen und pH-Wert-Messungen innerhalb lebender Zellen, Geweben und ganzen Organismen als Medium durchgeführt werden. Eine Fluoreszenz-Färbung von Zellen und Geweben durch Inkubation dieser Medien mit Ageladine A ist möglich. Vorteilhafterweise kann bei der Erfindung eine in vivo-oder in vitro-Inkubation von Zellen, Geweben oder kompletten Organismen als Medium in einer den fluoreszierenden pH-Wert-Indikator enthaltenden Lösung erfolgen. Durch eine derartige einfache, schnelle und biologische Färbung können beispielsweise saure Gewebsanteile (Verdauungsorgane) in lebenden Organismen markiert und unter dem Fluoreszenz-Mikroskop leicht erkannt werden. Ageladine A kann deshalb als äußerst intensiver Zellfarbstoff angewendet werden, der gleichzeitig als pH-Wert-Indikator pH-Wert-Änderungen reproduzierbar anzeigt. Qualitative pH-Wert-Anzeigen und quantitative pH-Wert-Messungen innerhalb lebender Systeme können durch die Erfindung stark vereinfacht werden.

Zusammenfassend zeigt das Verfahren nach der Erfindung mit einer Verwendung von Ageladine A als pH-Wert-Indikator folgende besondere Vorteile:
- Leichte Handhabbarkeit. Durch Vollsynthese steht der marine Naturstoff Ageladine A in großen Mengen zur Verfügung, ohne den Artenschutz zu gefährden.
- Große Empfindlichkeit von Ageladine A im physiologisch besonders interessanten Bereich zwischen pH6 und pH8.
- Die Fluoreszenz ist trotz UV-Bestrahlung. über Stunden stabil. Die Färbungen halten sich wenigstens über mehrere Tage.
- Kurzfristige Inkubation von Zellen und Geweben mit Ageladine A führt zu deutlicher und starker Fluoreszenz.
- Sogar intakte, lebende Organismen lassen sich mit Ageladine A färben, da es auf Grund seiner lipophilen Struktur (Bromierung) sehr schnell und weit in die Gewebe eindringt.
- Saure Bestandteile von Organismen lassen sich sofort nach Inkubation auf einen Blick im Fluoreszenz-Mikroskop erkennen, da die Fluoreszenz mit sinkendem pH-Wert stark zunimmt.

Weiter oben wurde bereits ausgeführt, dass sich verschiedene Störfaktoren ggfs. negativ auf die pH-Wert-Messungen auswirken können, wenn nur bei einer einzigen Anregungswellenlänge gemessen wird. Vorteilhaft kann deshalb auch das Verfahren nach der Erfindung unter Anwendung von Ageladine A als fluoreszierendem pH-Wert-Indikator modifiziert werden durch
- eine Fluoreszenzanregung bei zwei ausgewählten Wellenlängen
- Detektion der emittierten Fluoreszenzintensitäten
- Berechnung des Verhältnisses der Fluoreszenzintensitäten und
- Korrelation des Verhältnisses mit einer vorgegebenen Beziehung für solche Verhältnisse mit dem pH-Wert der Lösung.

### AUSFÜHRUNGSBEISPIELE

Das optische Messverfahren nach der Erfindung unter Anwendung von Ageladine A als fluoreszierendem pH-Wert-Indikator wird nachfolgend anhand der schematischen Figuren noch zum weiteren Verständnis der Erfindung näher erläutert. Dabei zeigt:
- FIGUR **1A**: die Strukturformel von Ageladine A,
- FIGUR 1**B**: die Protonierung und Stabilisierung von Ageladine A,
- FIGUR **2A,2B**: Fluoreszenz von mit Ageladine A gefärbten Zellen,
- FIGUR **3**: das Fluoreszenzintensitätsdiagramm von Ageladine A in Abhängigkeit de pH-Werts,
- FIGUR **4**: das Anregungsspektrum von Ageladine A in Abhängigkeit des pH-Werts,
- FIGUR **5**: Messungen des intrazellulären pH-Werts während der Änderung des extrazellulären pH-Werts,
- FIGUR **6A**: einen Organismus (Garnele),
- FIGUR **6B**: eine Lebendfärbung der Garnele mit Ageladine A und
- FIGUR 7: das Anregungsspektrum von Ageladine A in der Garnele.

Die Strukturformel von Ageladine A ist der FIGUR **1A** zu entnehmen. Die FIGUR **1B** zeigt die Protonierung der Guanidingruppe in Ageladine A und die Stabilisierung der positiven Ladung über die gesamte Struktur. Die rechte Struktur beinhaltet eine starre und planare Anordnung, die unter anderem für die Fluoreszenz verantwortlich ist.

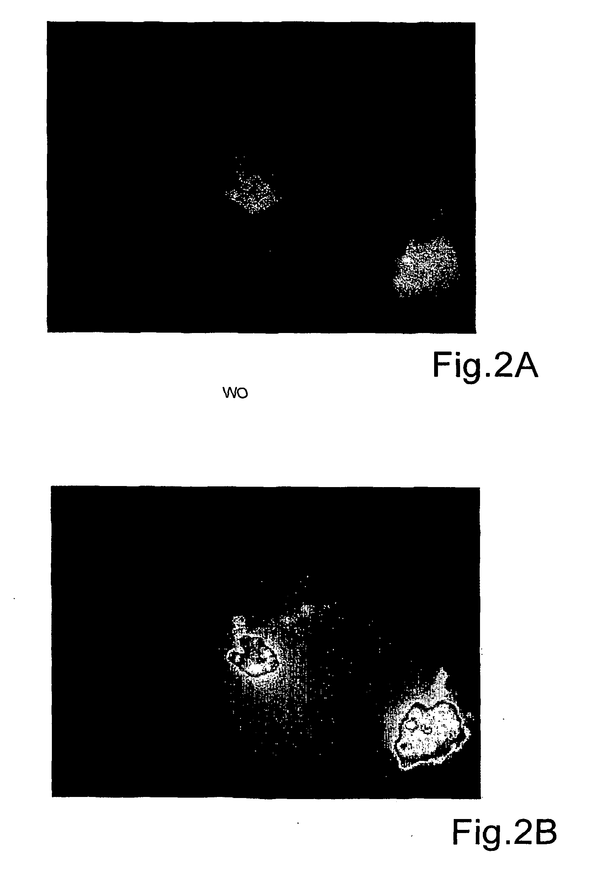

Die FIGUREN **2A****,** **1B** zeigen in 400facher Vergrößerung Aufnahmen von mit Ageladine A inkubierten und dadurch gefärbten lebenden Zellen (PC-12-Zellen, Ratte, hinterlegt bei DSZM). Die Anregung erfolgte mit UV-Licht mit einer Wellenlänge von 380 nm.

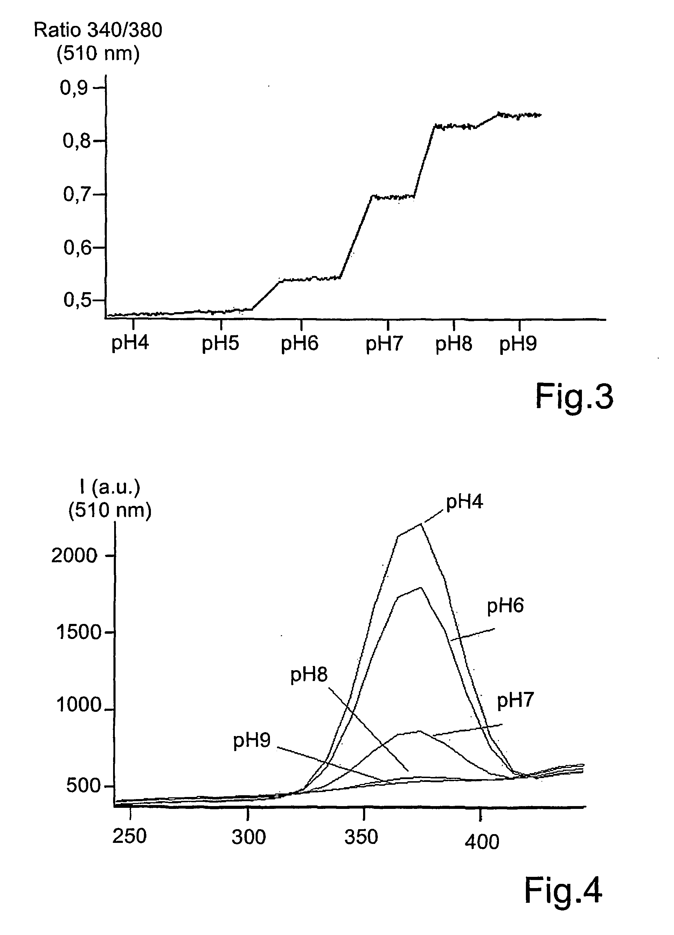

Die **FIGUR 3** zeigt den Fluoreszenz-Ratio-Wert (gemessen bei einer Fluoreszenzemission bei 510 nm) der Anregungswellenlängen 340 nm / 380 nm bei verschiedenen pH-Werten für eine Zugabe von 20 µM Ageladine A in einen physiologischen Puffer. Deutlich ist der messbare große pH-Wert-Bereich mit einer guten Zuordenbarkeit der gemessenen Ratio-Werte zu den einzelnen pH-Werten zu erkennen.

Die **FIGUR 4** zeigt das Anregungsspektrum (emittierte Fluoreszenzintensität über der Anregungswellenlänge in nm) von Ageladine A bei verschiedenen pH-Werten der Lösung. Die Fluoreszenzintensität wurde bei 510 nm gemessen. Zu erkennen ist die große pH-Wert-Empfindlichkeit von Ageladine A im physiologisch relevanten Bereich um pH7 herum.

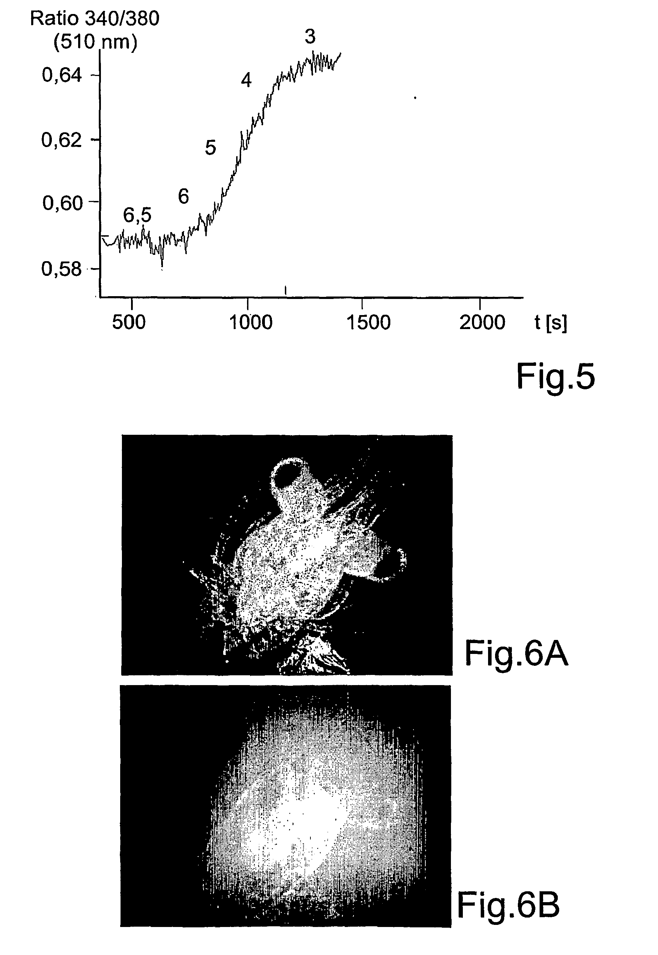

Die **FIGUR 5** zeigt das Messprotokoll einer Messung des intrazellulären pH-Werts von PC12 Zellen mittels Ageladine A bei Änderung des äußeren pH-Werts der Lösung. Aufgetragen ist der Fluoreszenz-Ratio-Wert (340 nm/380 nm) über der Zeit (time in s). Die verschiedenen pH-Werte der Pufferlösung sind als Parameter am Graphen markiert. Deutlich ist zu erkennen, dass die Fluoreszenzemission dem extrazellulären Ansteigen des pH-Wertes der Lösung in einem weiten Bereich (pH6,5 bis pH3) folgt. Die Änderung des intrazellulären pH-Werts beträgt jedoch nur einige Zehntel um pH7 herum, wodurch eine sehr hohe Messauflösung des pH-Wert-Indikators gegeben ist.

Die **FIGUR 6A** zeigt in 100facher Vergrößerung ein mikroskopisches Durchlichtbild eines lebenden Organismus, hier einer Garnele (*Palaemonetes argentinus*) von oben. Die **FIGUR 6B** zeigt das Fluoreszenzbild dieser Garnele, nachdem diese mit Ageladine A inkubiert und mit UV-Licht bestrahlt wurde. Aufgrund der Intensität der emittierten Fluoreszenz ist zu erkennen, dass saure Strukturen (Mund-Verdauungsbereich) in der Garnele gefärbt wurden.

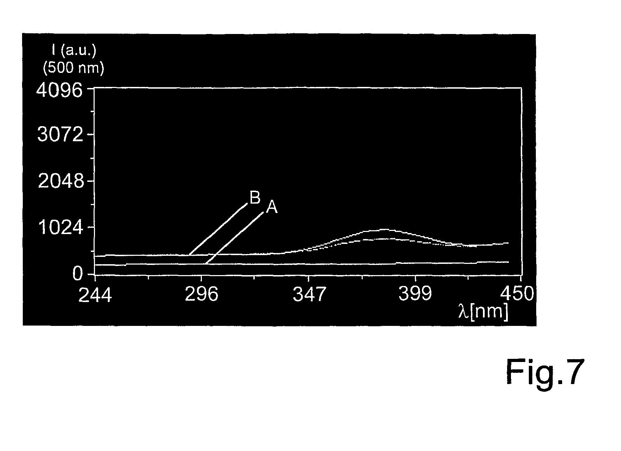

Die **FIGUR 7** zeigt das Anregungsspektrum (emittierte Fluoreszenzintensität über Anregungswellenlänge) von Ageladine A in der Garnele im Mund-Verdauungsbereich, gemessen bei 510nm Fluoreszenzemission. Die grüne Kurve (unterste, gerade Kurve) zeigt Fluoreszenz-Intensitätswerte innerhalb der Pufferlösung außerhalb der Garnele an, die rote und die blaue Kurve (mittlere und obere gekrümmte Kurve) zeigen Fluoreszenz-Intensitätswerte innerhalb der Garnele und damit in einfacher Weise leicht erkenn- und detektierbare saure Bestandteile der Garnele an.

## Patentansprüche

1. Optisches Messverfahren zur Ermittlung des pH-Werts eines Mediums durch
• Zugabe eines fluoreszierenden pH-Wert-Indikators in das Medium,
• Fluoreszenzanregung des pH-Wert-Indikators durch Bestrahlung des pH-Wert-Indikators mit Licht zumindest einer ausgewählten Wellenlänge und
• Detektion der emittierten Fluoreszenzintensität des pH-Wert-Indikators als Maß für den pH-Wert des Mediums,
**GEKENNZEICHNET DURCH**
die Verwendung eines fluoreszierenden pH-Wert-Indikators auf Basis von natürlich gewonnenem oder synthetisiertem Ageladine A mit einer Fluoreszenz im Grünbereich zwischen 490 nm und 575 nm Wellenlänge bei einer Fluoreszenzanregung **durch** Bestrahlung mit Licht im UV-Bereich zwischen 315 und 400 nm Wellenlänge und einer hohen Empfindlichkeit der emittierten Fluoreszenzintensität im physiologisch besonders relevanten Bereich zwischen pH6 und pH8, wobei pH-Wert-Änderungen des zu untersuchenden Mediums zwischen pH4 und pH9 **durch** eine veränderliche Fluoreszenzintensität in der Weise optisch angezeigt werden, dass mit sinkendem pH-Wert die emittierte Fluoreszenzintensität ansteigt.

2. Optisches Messverfahren nach Anspruch 1,
**GEKENNZEICHNET DURCH**
eine in vivo- oder in vitro-Inkubation von Zellen, Geweben oder kompletten Organismen als Medium in einer den fluoreszierenden pH-Wert-Indikator enthaltenden Lösung.

3. Optisches Messverfahren nach Anspruch 2,
**GEKENNZEICHNET DURCH**
eine Betrachtung des inkubierten Mediums in einem Fluoreszenz-Mikroskop,

4. Optisches Messverfahren nach einem der Ansprüche 1 bis 3, **GEKENNZEICHNET DURCH**
• eine Fluoreszenzanregung bei zwei ausgewählten Wellenlängen
• Detektion der emittierten Fluoreszenzintensitäten
• Berechnung des Verhältnisses der Fluoreszenzintensitäten und
• Korrelation des Verhältnisses mit einer vorgegebenen Beziehung für solche Verhältnisse mit dem pH-Wert der Lösung.

## Claims

1. Optical measuring method for determining the pH value of a medium through
• the addition of a fluorescing pH value indicator into the medium
• fluorescence activation of the pH value indicator through exposing the pH value indicator to light at least one selected wavelength and
• detection of the emitted fluorescence intensity of the pH value indicator as a measure of the pH value of the medium
**characterised by**
the use of a fluorescing pH value indicator based on naturally obtained or synthesised ageladine A with fluorescence activation through exposure to light in a UV range of between 315 nm and 400 nm wavelength and a high sensitivity of the emitted fluorescence intensity in the physiologically particularly relevant range of between pH6 and pH8, whereby pH value changes in the medium to be examined between pH4 and pH9 are optically shown through a changing fluorescence intensity in that with a decreasing pH value the emitted fluorescence intensity increases.

2. Optical measuring method in accordance with claim 1,
**characterised by**
in-vivo or in-vitro incubation of cells, tissues or complete organisms as the medium in a solution containing the fluorescing pH value indicator.

3. Optical measuring method in accordance with claim 2,
**characterised by**
examining the incubated medium in a fluorescence microscope.

4. Optical measuring method in accordance with any one of claims 1 to 3
**characterised by**
• a fluorescence activation at two selected wavelengths
• detection of the emitted fluorescence intensities
• calculation of the ratios of the fluorescence intensities and
• correlation of these ratios with a predetermined relationship of such ratios with the pH value of the solution.

## Revendications

1. Procédé de mesure optique pour la détermination du pH d'un milieu par :
• addition d'un indicateur de pH fluorescent dans le milieu,
• stimulation de fluorescence de l'indicateur de pH par irradiation de l'indicateur de pH avec de la lumière d'au moins une longueur d'ondes sélectionnée et
• détection de l'intensité de fluorescence émise de l'indicateur de pH comme référence pour le pH du milieu,
**caractérisé par**
l'utilisation d'un indicateur de pH fluorescent sur la base d'agéladine A obtenue naturellement ou synthétisée avec une excitation de fluorescence par irradiation avec de la lumière dans la plage des UV comprise entre 315 nm et 400 nm de longueur d'onde et une sensibilité élevée de l'intensité de fluorescence émise dans la plage particulièrement importante au plan physiologique comprise entre pH6 et pH8, des variations du pH du milieu à étudier entre pH4 et pH9 étant affichées visuellement par une intensité de fluorescence variable en ce sens que l'intensité de fluorescence émise augmente avec l'abaissement du pH.

2. Procédé de mesure optique selon la revendication 1,
**caractérisé par**
une incubation in vivo ou in vitro de cellules, tissus ou organismes complets comme milieu dans une solution contenant l'indicateur de pH fluorescent.

3. Procédé de mesure optique selon la revendication 2,
**caractérisé par**
un examen du milieu incubé dans un microscope à fluorescence.

4. Procédé de mesure optique selon l'une quelconque des revendications 1 à 3,
**caractérisé par**
• une excitation de fluorescence avec deux longueurs d'ondes choisies,
• détection des intensités de fluorescence émises
• calcul du rapport des intensités de fluorescence et
• corrélation du rapport avec une relation prédéfinie pour de tels rapports avec le pH de la solution.
